# EUROPÄISCHE PATENTSCHRIFT

(11) **EP 2 818 104 B1**
(45) Veröffentlichungstag und Bekanntmachung des Hinweises auf die Patenterteilung: **06.01.2016**
(21) Anmeldenummer: 14170181.3
(22) Anmeldetag: 28.05.2014
(51) Int. Cl.: A61B 5/042, A61B 18/14

(54) **Katheter und Verfahren zur Herstellung desselben**
Catheter and method for producing same
Cathéter et méthode de sa fabrication

(30) Priorität: 25.06.2013 US 201361838874 P
(43) Veröffentlichungstag der Anmeldung: 31.12.2014
(73) Patentinhaber: VascoMed GmbH, 79589 Binzen (DE)
(72) Erfinder: Geistert, Wolfgang, 79618 Rheinfelden (DE); Schulze, Daniel, 8004 Zürich (CH)
(74) Vertreter: Galander, Marcus

(56) Entgegenhaltungen:
- EP-A1- 0 779 059
- DE-A1- 4 211 852
- US-A1- 2010 217 257

## Beschreibung

Die Erfindung betrifft einen Katheter, insbesondere Herz- oder Gefäßkatheter und ein Verfahren zur Herstellung desselben.

Herz- bzw. Gefäßkatheteruntersuchungen sind minimalinvasive medizinische Untersuchungen des Herzens mit einem Katheter, der über venöse oder arterielle Adern an der Leiste, der Ellenbeuge oder über das Handgelenk eingeführt wird. Je nach Untersuchungsziel werden unterschiedliche Stellen im Herzen oder der Herzkranzgefäße oder der peripheren Gefäße angesteuert. Im Rahmen der Katheteruntersuchung können beispielsweise Messungen der elektrischen Aktivitäten bei Erregungsstörungen durchgeführt werden und gegebenenfalls besteht beispielsweise die Möglichkeit der direkten Behandlung durch Ablation von muskulären oder nervalen Erregerbahnen. Der Katheter weist zu solchen Zwecken mindestens eine, in der Regel jedoch zwei oder mehr an einer Außenoberfläche des Katheters angeordnete Elektroden auf, mit denen die Messung beziehungsweise Behandlung durchgeführt werden kann. Je nach konkretem Diagnostik- und/oder Behandlungszweck gibt es eine Vielzahl angepasster Varianten derartiger Katheter; beispielsweise zeigt US 5,239,999 A einen sogenannten Ablationskatheter.

Ferner ist aus EP 0 779 059 A1 ein Katheter mit plattenförmiger Anordnung bekannt.

Die klassische Art und Weise eine Elektrode auf einem Katheter herzustellen und zu kontaktieren ist, diese aus einem Metallteil zum Beispiel Ring oder Plättchen herzustellen, welches dann mittels Löten, Lasern oder Widerstandsschweißen mit einem Zuleitungsdraht verbunden wird. Anschließend wird der Zuleitungsdraht durch ein Loch in der Katheterwandung in ein inneres Lumen des Katheterschafts eingefädelt und zu dessen proximalen Ende geführt. Das Metallteil wird distal an der vorgesehenen Stelle des Katheterschafts durch Kleben, Krimpen, Klemmen oder dergleichen angebracht. Der Zuleitungsdraht wird schließlich an seinem anderen Ende mit einem Steckverbinder verbunden. Die klassische Herstellungstechnologie ist somit relativ aufwendig und arbeitsintensiv.

US 5,239,999 A beschreibt einen Katheter dessen distales Ende eine helikale Wicklung aufweist, auf der die Elektroden nebst zugehöriger Leiterbahnen untergebracht sind. Diese Wicklung wird distal verankert und um den Katheterschaft gewickelt.

Ein oder mehrere bekannte Nachteile des Standes der Technik lassen sich mit Hilfe des erfindungsgemäßen Katheters, insbesondere Herz- oder Gefäßkatheters, beheben oder zumindest mindern. Der Katheter weist eine Folienverbundstruktur auf, die zumindest umfasst:
(i) eine Polymerfolie, welche so geformt ist, dass eine bezüglich des Katheters innenliegende erste Polymerfolienlage und eine bezüglich des Katheters außenliegende zweite Polymerfolienlage entstehen;
(ii) eine oder mehr zumindest teilweise an einer Außenoberfläche der Folienverbundstruktur angeordnete Elektroden; und
(iii) eine Leiterstruktur, die Leiterbahnen zum elektrischen Anschluss der Elektroden umfasst und die zumindest teilweise zwischen der ersten und zweiten Polymerfolienlage angeordnet ist.

Der Erfindung liegt die Erkenntnis zu Grunde, dass sich ein mit Elektroden ausgestatteter Katheter besonders einfach und zuverlässig dadurch herstellen lässt, dass die gesamte Elektrodenkonfiguration, also Elektroden nebst zugehöriger Leiterbahnen, Teil einer flexiblen, in an sich bekannter Leiterplattentechnologie hergestellten Folienverbundstruktur ist, wobei die Folienverbundstruktur gleichzeitig zumindest Teile des Lumens des Katheterschafts bildet. Mit anderen Worten, die Folienverbundstruktur enthält die zur elektrischen Schaltung der Elektroden notwendigen Leiterbahnen, als auch die Elektroden selbst. Dabei weist die Verbundstruktur zumindest eine innenliegende und außenliegende Lage auf. Die Elektroden sind auf der äußeren Polymerlage angeordnet, um ihrer bestimmungsgemäßen Verwendung nachzukommen. Die Folienverbundstruktur bildet damit selbst die Katheterwandung in diesem Bereich des Katheters und bestimmt so das Lumen des Katheters zumindest mit. Auf diese Weise wird ein sehr kompakter Aufbau des Katheterschafts ermöglicht, so dass im Lumen des Katheters genügend Bauraum für Führungsdrähte, Kanülen, Sensoren etc. ist, der eine verbesserte Anpassung des Katheters an den jeweiligen Verwendungszweck erlaubt. Ferner ist durch die Integration von Leiterbahnen und Elektroden in die Folienverbundstruktur eine verbesserte mechanische Belastbarkeit des Katheters gegeben. Schließlich ist das Fertigungsverfahren des Katheters wesentlich vereinfacht und weniger fehleranfällig, weil letztendlich nur noch der vorgefertigte Folienverbund zum Aufbau der Elektrodenkonfiguration an entsprechender Stelle platziert und mit den weiteren Bestandteilen des Katheters verbunden werden muss.

Vorzugsweise ist die Polymerfolie aus einem thermoplastischen Kunststoff geformt, insbesondere einem Flüssigkristallpolymer (LCP). Flüssigkristallpolymere zeichnen sich durch für die Anwendungszwecke besonders geeignete mechanische Eigenschaften aus. Alternativ können die Polymerfolien auch aus Polyurethan, Polyamid oder Polyether-Blockamiden (PEBA) bestehen. Die vorgeschlagenen thermoplastischen Kunststoffe haben den Vorteil, dass sie durch eine direkte Material-Material-Verbindung (Verschweißen, Verschmelzen, Ineinanderfließen) ohne die Verwendung von zusätzlichen Klebstoffen stoffschlüssig miteinander verbunden werden können.

Die Folienverbundstruktur bildet aufgerollt ein Lumen des Katheters, wobei die sich überlappenden Bereiche der Folienverbundstruktur durch eine stoffschlüssige Fügeverbindung (direkte Material-Material-Verbindung) dichtend miteinander verbunden sind.

Ein weiterer Aspekt der Erfindung betrifft ein dazugehöriges Verfahren zur Herstellung des Katheters, das die Schritte umfasst:
a) Bereitstellen einer Folienverbundstruktur, die zumindest (i) eine Polymerfolie, (ii) eine oder mehr an einer Außenoberfläche der Folienverbundstruktur angeordnete Elektroden und (iii) eine Leiterstruktur mit Leiterbahnen zum elektrischen Anschluss der Elektroden umfasst; und
b) Formen (z.B. Aufrollen) der Folienverbundstruktur, derart, dass sich diese so überlappt, dass sie ein Lumen des Katheters bildet, wobei eine bezüglich des Katheters innenliegende Polymerfolienlage und eine bezüglich des Katheters außenliegende Polymerfolienlage entstehen, sich die Elektroden zumindest teilweise auf der Außenfläche befinden und die sich überlappenden Teile der Folienverbundstruktur durch stoffschlüssiges Fügen dichtend miteinander verbunden werden.

Das erfindungsgemäße Herstellungsverfahren sieht demnach vor, dass die zuvor beschriebene Folienverbundstruktur zur Ausbildung von zumindest Teilabschnitten des Katheterschafts des Katheters aufgerollt oder aufgewickelt wird und überlappende Bereiche der Folienverbundstruktur über ein Verbindungsverfahren dichtend miteinander verbunden werden. Die Folienverbundstruktur bildet dann quasi die Wandung des Katheterschafts in diesem Bereich. Im Prinzip eignen sich alle gängigen stoffschlüssigen Verbindungsverfahren für thermoplastische Polymere. Von besonderem Vorteil ist es jedoch, wenn das Fügen durch Schmelzschweißen oder Anlösen und ohne nichtflüchtigen Fügezusatzstoff (wie Klebstoff) erfolgt. Gerade für den medizintechnischen Einsatz ist ein Verzicht auf nichtflüchtige Fügezusatzstoffe von besonderer Bedeutung, denn es muss sichergestellt sein, dass diese Zusatzstoffe keine unerwünschten Komplikationen bei intrakorporalem Körperkontakt hervorrufen können. Die dazu notwendigen Untersuchungen sind jedoch sehr zeit- und kostenintensiv. Außerdem kann ein solcher Zusatzstoff den Katheter versteifen, so dass dieser nicht die erwünschte Flexibilität aufweist.

Beim Schmelzschweißen werden die Bereiche der Folienverbundstruktur, die miteinander verbunden werden sollen, durch Wärme erweicht, zusammengepresst, miteinander verbunden und wieder abgekühlt.

Zum Schmelzschweißen ohne Fügezusatzstoff kann die Wärmeenergie lokal durch eine Wärmequelle (beispielsweise beheizte Form, Heizbacken) eingebracht werden. Vorteilhafterweise kann das Aufheizen der Folienverbundstruktur auch durch auf- oder eingebrachte Leiterbahnen realisiert werden, welche an den aufzuschmelzenden Stellen angeordnet sind und durch welche ein Strom geschickt wird, der in der Leiterbahn in Wärme umgesetzt und so die Trägerfolie aufgeschmolzen wird. Mit anderen Worten, die Folienverbundstruktur weist vorzugsweise auf- oder eingebrachte Leiterbahnen auf, die an den durch Schmelzschweißen aufzuschmelzenden Bereichen der Folienverbundstruktur angeordnet sind. Diese Leiterbahnen dienen zum elektrischen Beheizen der aufzuschmelzenden Bereiche. Schließlich kann die zum Aufschmelzen notwendige Wärmeenergie auch durch Reibung (Ultraschallschweißen) erzeugt werden. Dabei werden die zu verbindenden Teile des Verbunds so schnell (mit Ultraschallgeschwindigkeit) unter Druck aneinander gerieben, dass die Oberfläche der Trägerfolie durch die entstehende Reibungswärme aufschmilzt, die sich überlappenden Teile der Trägerfolie ineinander fließen und so miteinander verbinden. Dabei ist es von Vorteil, wenn die Oberfläche der Trägerfolie zumindest an den zu verbindenen Stellen rau ist. Dies kann beispielsweise durch Behandlung des betreffenden Oberflächenbereichs mit einer Ätzlösung erreicht werden. Zum Beispiel kann mit Toray's TPE3000, einer bekannten Ätzlösung zum Nassätzen von Polyimid, die Oberfläche von LCP angeätzt und somit aufgeraut werden.

Für das Aufschmelzen von LCP sind in der Regel Temperaturen im Bereich von 250 bis 310°C, meist um 290°C erforderlich. Für Polyurethan und PEBA werden Temperaturen im Bereich 140 bis 200°C benötigt.

Alternativ kann das Fügen durch Anlösen von zu verbindenden Bereichen der Folienverbundstruktur mit einem Lösungsmittel, Zusammenpressen der angelösten Bereiche und vollständiges Entfernen des Lösungsmittels erfolgen. Auch hier steht somit im Vordergrund, dass der Verbindungsbereich keine weiteren nichtflüchtigen Fügezusatzstoffe enthält, es sich demnach nach wie vor nur um das Material handelt, aus dem die Folienverbundstruktur besteht.

Lösungsmittel, welche zur Verbindung der sich überlappenden Teile der Folienverbundstruktur verwendet werden können, sind im Falle von LCP beispielsweise fluorierte Phenole, wie Pentafluorphenol, Tetrafluorphenol oder 3,5-Bistrifluormetylphenol. Im Falle von Polyurethan und PEBA können als Lösungsmittel Dimethylformamid, Tetrahydrofuran oder Pyridin verwendet werden.

Eine Schichtdicke der Polymerfolie liegt vorzugsweise im Bereich von 10 bis 100 µm

Die Leiterstruktur ist vorzugsweise zur Isolation und zum Schutz in eine Klebefolie eingebettet und durch eine zweite Polymerfolie abgedeckt. Auch diese Klebefolie kann aus einem Flüssigkristallpolymer geformt sein, dessen Schmelzpunkt jedoch etwas niedriger gewählt ist, als der Schmelzpunkt der beiden zu verbindenden Polymerfolien. Alternativ kann die Leiterstruktur durch eine Lackschicht abgedeckt sein.

Bei der erfindungsgemäßen Elektrodenkonfiguration wird demnach eine thermoplastische, biokompatible Trägerfolie (Polymerfolie) aus den genannten Materialien verwendet, auf der mittels bekannter Leiterplattentechnologie im flachen Zustand metallische Elektrodenflächen aufgebracht, ausgeätzt oder abgeschieden werden. Die Leiterbahnen der Leiterstruktur werden vorzugsweise aus Kupfer geformt. Die Elektroden bestehen vorzugsweise aus einem hochleitfähigen, biokompatiblen Metallwerkstoff, wie zum Beispiel Gold, Platin, Platin-Iridium-Legierungen oder Palladium-Legierungen oder weisen zumindest eine Beschichtung aus diesem Metallwerkstoff auf. In letzterem Fall wird insbesondere Kupfer als Basismaterial für die Beschichtung verwendet.

Die Elektrodenkonfiguration wird in die gewünschte räumliche Form gebracht, in der Regel als Zylinder aufgerollt, so dass sich zumindest ein Teil der Elektrodenflächen auf der Außenseite der räumlichen Form befinden und der Folienverbund überlappt. Die sich überlappenden Bereiche des Folienverbunds werden, wie zuvor bereits beschrieben, miteinander verbunden. Die Elektroden sind vorzugsweise als Ringelektroden ausgebildet.

Weitere bevorzugte Ausgestaltungen der Erfindung ergeben sich aus den Unteransprüchen beziehungsweise der weiteren Beschreibung.

Die Erfindung wird nachfolgend anhand von Figuren und einem dazugehörigen Ausführungsbeispiel erläutert. Es zeigen:
- Fig. 1: eine schematische Darstellung eines Zwischenprodukts zur Herstellung eines Katheters mit einer distal angeordneten Folienverbundstruktur;
- Fig. 2: einen Längsschnitt durch den erfindungsgemäßen Katheter im Bereich der Folienverbundstruktur;
- Fig. 3: ein Querschnitt durch den erfindungsgemäßen Katheter im Bereich der Folienverbundstruktur;
- Fig. 4: eine schematische Darstellung der aufgerollten Folienverbundstruktur; und
- Fig. 5: eine Draufsicht auf das distale Ende eines erfindungsgemäßen Katheters.

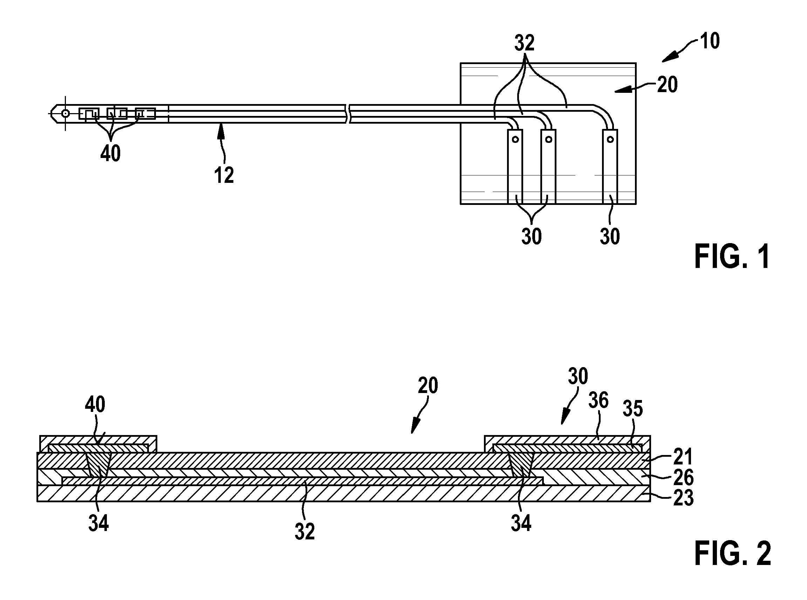

Figur 1 zeigt - schematisiert - eine Draufsicht auf ein Zwischenprodukt des Fertigungsprozesses eines Katheters. Der rechte Teil von Figur 1 zeigt den Bereich des distalen Katheterschafts 10, der später die Elektrodenkonfiguration tragen soll. In einem, hier im abgerollten Zustand dargestellten Bereich des Katheterschafts 10 befindet sich eine Folienverbundstruktur 20. Die Folienverbundstruktur 20 weist vorliegend insgesamt drei Elektroden 30 auf, die über Leiterbahnen 32 elektrisch angebunden sind. Die Leiterbahnen 32 werden zum proximalen Ende des Katheterschafts 10 geführt (linker Teil von Figur 1) und mit geeigneten Kontaktflächen 40 verbunden. Auch der proximalen Teil 12 des Katheterschafts 10 kann aus der Folienverbundstruktur 20 geformt sein. Mit anderen Worten, die Folienverbundstruktur 20 kann sich über den gesamten Bereich der Elektrodenkonfiguration nebst Leiterbahnen 32 bis hin zu den proximalen Anschlussstellen der Leiterbahnen 32 an die weiteren Bauelemente der elektrischen Schaltung (Steckverbindungen) erstrecken.

Figur 2 illustriert - wiederum rein schematisch - einen Längsschnitt durch eine Ausführungsform der Folienverbundstruktur 20 mit einer Elektrode 30, einer Leitbahn 32 und einer proximalen Kontaktfläche 40. Gemäß diesem Ausführungsbeispiel umfasst die Folienverbundstruktur 20 eine erste, die äußere Oberfläche des Katheterschafts bildende Polymerfolie 21 und eine zweite, abdeckende Polymerfolie 23. Zwischen den beiden Polymerfolien 21, 23 befindet sich eine Klebeschicht 26, in die eine Leiterbahn 32 integriert ist und die die beiden Polymerfolien 21, 23 verbindet.

Die Leiterbahnen 32 ist mittels einer Durchkontaktierung 34 mit der außen auf der ersten Polymerfolie 21 liegenden Elektrode 30 verbunden. Die Elektrode 30 ist vorliegend zweilagig aufgebaut, mit einer unteren Lage 35 aus Kupfer und einer darauf aufgetragenen, die eigentliche Elektrodenfläche vorgebenden oberen Lage 36 aus Gold. Auch die Leiterbahn 32 besteht vorliegend aus Kupfer.

Die beiden Polymerfolien 21, 23, als auch die Klebefolie 26, werden gemäß dem Ausführungsbeispiel auf Basis eines Flüssigkristallpolymers hergestellt, wobei die Klebefolie 26 so modifiziert ist, dass sie bei einer niedrigeren Temperatur erweicht und so ein Laminieren der beiden Polymerfolien 21, 23 erlaubt. Alle drei Folienlagen 21, 23 und 26 haben vorliegend eine Schichtdicke von etwa 25 µm. Die Leiterbahn 32 besitzt eine Schichtdicke von etwa 16 µm. Die untere Lage 35 der Elektrode 30 weist eine Schichtdicke von etwa 5 bis 7 µm auf und die obere Lage 36 aus Gold eine Schichtdicke von etwa 3 bis 4 µm.

In einer vereinfachten Ausführungsform, kann die Folienverbundstruktur auch nur aus einer Polymerfolie 21 bestehen und die zweite Polymerfolie 23 sowie die Klebeschicht 26 weggelassen sein. Bei einer solchen vereinfachten Ausführungsform kann die Leiterbahn 32 auf der gleichen Seite der Polymerfolie 21 wie die Elektrode 30 angeordnet sein. Alternativ kann die Leiterbahn 32 auf der Rückseite der Polymerfolie 21 angeordnet und über eine Durchkontaktierung 34 mit der auf der Vorderseite der Polymerfolie 21 angeordneten Elektrode 30 verbunden sein. Ebenso kann die proximale Kontaktfläche 40 auf der gleichen Seite der Polymerfolie 21 wie die Leitbahn 32 oder verbunden über eine Durchkontaktierung 34 auf der anderen Seite der Polymerfolie 21 angeordnet sein.

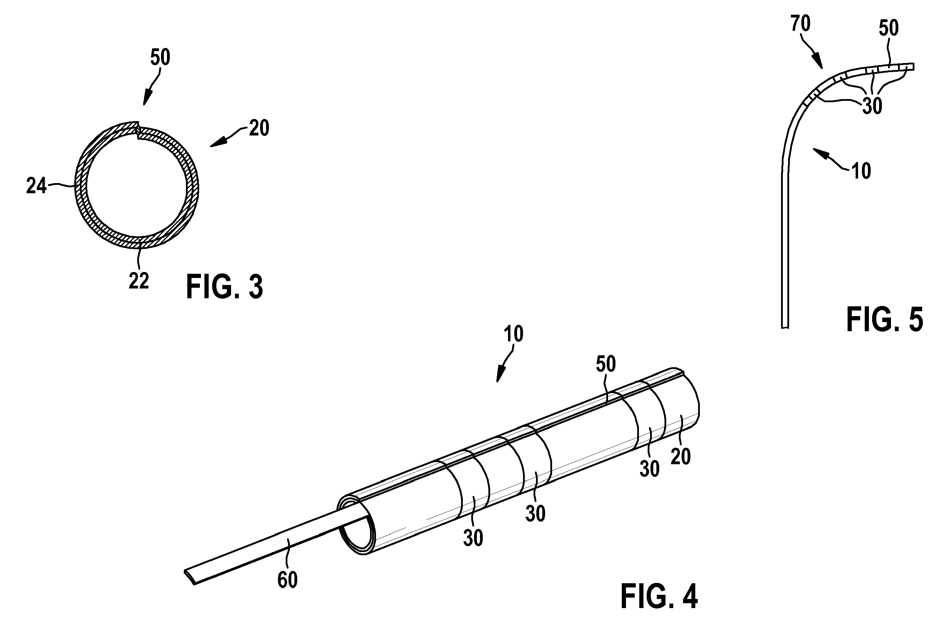

Figur 3 zeigt einen Querschnitt durch den Katheterschaft des Katheters im Bereich der Folienverbundstruktur 20. Die Verbundstruktur 20 wird so aufgerollt, dass sich Bereiche überlappen. Im vorliegenden Beispiel sind zwei Windungen ausgeführt, so dass eine innere Polymerfolienlage 22 und eine äußere Polymerfolienlage 24 entstehen und der Überlappungsbereich eine volle Windung beträgt. Durch ein geeignetes stoffschlüssiges Fügeverfahren werden die beiden Polymerfolienlagen 22, 24 in zumindest Teilen des Überlappungsbereiches, insbesondere aber an der Kante 50 der äußeren Polymerfolienlage 24, dichtend miteinander verbunden.

Die Verbindung wird gemäß dem vorliegenden Beispiel durch ein Schmelzschweißverfahren ohne Einsatz eines weiteren Fügezusatzstoffs hergestellt.

Figur 4 zeigt einen Teil des Katheterschafts 10 des Katheters im Bereich der Folienverbundstruktur 20. Wie ersichtlich sind die Elektroden 30 als Ringelektroden ausgebildet. Die hier nicht sichtbaren Leiterbahnen 32 liegen im Inneren der Folienverbundstruktur 20 und werden über eine Verlängerung 60 fortgeführt. Zu erkennen ist, dass innerhalb des Lumens des Katheterschafts genügend Platz für weitere Katheterbauteile verbleibt.

Figur 5 zeigt nochmals das distale Ende 70 des Katheterschafts 10 des erfindungsgemäßen Katheters. Zu erkennen ist die Folienverbundstruktur 20 mit ihren integrierten Ringelektroden 30. Das distale Ende 70 des Katheterschafts kann beispielsweise durch einen thermischen Prozess in eine gebogene Form gebracht werden.

## Patentansprüche

1. Katheter mit einer Folienverbundstruktur (20), die zumindest umfasst:
(i) eine Polymerfolie (21), welche so geformt ist, dass eine bezüglich des Katheters innenliegende erste Polymerfolienlage (22) und eine bezüglich des Katheters außenliegende zweite Polymerfolienlage (24) entstehen;
(ii) eine oder mehr zumindest teilweise an einer Außenoberfläche der Folienverbundstruktur (20) angeordnete Elektroden (30); und
(iii) eine Leiterstruktur, die Leiterbahnen (32) zum elektrischen Anschluss der Elektroden (30) umfasst und die zumindest teilweise zwischen der ersten und zweiten Polymerfolienlage (22, 24) angeordnet ist.
**dadurch gekennzeichnet, dass** die Folienverbundstruktur (20) aufgerollt ein Lumen des Katheters bildet, wobei überlappende Bereiche der Folienverbundstruktur (20) durch eine stoffschlüssige Fügeverbindung dichtend miteinander verbunden sind.

2. Katheter nach Anspruch 1, bei dem die Fügeverbindung keinen Fügezusatzstoff enthält.

3. Katheter nach einem der vorhergehenden Ansprüche, bei dem die Polymerfolie (21) aus einem thermoplastischen Kunststoff, insbesondere einem Flüssigkristallpolymer, einem Polyurethan, einem Polyamid oder einem Polyether-Blockamid geformt ist.

4. Katheter nach einen der vorhergehenden Ansprüche, bei dem eine Schichtdicke der Polymerfolie (21) im Bereich von 10 bis 100 µm liegt.

5. Katheter nach einem der vorhergehenden Ansprüche, bei dem (i) die Folienverbundstruktur (20) eine zweite Polymerfolie (23) umfasst und die Leiterstruktur in eine die erste Polymerfolie (21) und die zweite Polymerfolie (23) verbindende Klebefolie (26) eingebettet ist oder (ii) die Leiterstruktur mit einer Lackschicht abgedeckt ist.

6. Katheter nach einem der vorhergehenden Ansprüche, bei dem die Leiterbahnen (32) aus Kupfer sind.

7. Katheter nach. einem der vorhergehenden Ansprüche, bei dem die Elektroden (30) aus einem hochleitfähigen, biokompatiblen Metallwerkstoff, insbesondere Gold, Platin, Platin-Iridium-Legierungen oder Palladium-Legierungen, bestehen oder eine Beschichtung aus diesem Metallwerkstoff aufweisen.

8. Katheter nach einem der vorhergehenden Ansprüche, bei dem wenigstens eine Elektrode (30) als Ringelektrode ausgebildet ist.

9. Verfahren zur Herstellung eines Katheters, umfassend die Schritte:
a) Bereitstellen einer Folienverbundstruktur (20), die zumindest (i) eine Polymerfolie (21), (ii) eine oder mehr an einer Außenoberfläche der Folienverbundstruktur (20) angeordnete Elektroden (30) und (iii) eine Leiterstruktur mit Leiterbahnen (32) zum elektrischen Anschluss der Elektroden (30) umfasst; und
b) Formen der Folienverbundstruktur (20), derart, dass sich diese so überlappt, dass sie ein Lumen des Katheters bildet, wobei eine bezüglich des Katheters innenliegende Polymerfolienlage (22) und eine bezüglich des Katheters außenliegende Polymerfolienlage (24) entstehen, sich die Elektroden zumindest teilweise auf der Außenfläche befinden und die sich überlappenden Teile der Folienverbundstruktur (20) durch stoffschlüssiges Fügen dichtend miteinander verbunden werden.

10. Verfahren nach Anspruch 9, bei dem das stoffschlüssige Fügen durch Schmelzschweißen und ohne Fügezusatzstoff erfolgt.

11. Verfahren nach Anspruch 10, bei dem das stoffschlüssige Fügen durch lokale Einbringung von Wärmeenergie mittels einer Wärmequelle erfolgt.

12. Verfahren nach Anspruch 10, bei dem das stoffschlüssige Fügen durch Ultraschallschweißen erfolgt.

13. Verfahren nach Anspruch 10, bei dem die Folienverbundstruktur (20) auf- oder eingebrachte Leiterbahnen aufweist, die an den durch Schmelzschweißen aufzuschmelzenden Bereiche der Folienverbundstruktur (20) angeordnet sind, und bei dem diese Leiterbahnen zum elektrischen Beheizen der aufzuschmelzenden Bereiche dienen.

14. Verfahren nach Anspruch 9, bei dem das stoffschlüssige Fügen durch Anlösen von zu verbindenden Bereichen der Folienverbundstruktur (20) mit einem Lösungsmittel, Zusammenpressen der angelösten Bereiche und vollständiges Entfernen des Lösungsmittels erfolgt.

## Claims

1. Catheter having a foil composite structure (20), which at least comprises:
(i) a polymer foil (21), which is shaped such that an internal, relative to the catheter, first polymer foil layer (22) and an external, relative to the cathether, second polymer foil layer (24) are produced;
(ii) one or more electrodes (30), which are disposed at least partially on an outer surface of the foil composite structure (20); and
(iii) a conductor structure, which includes conductor tracks (32) for the electrical connection of the electrodes (30) and is disposed at least partially between the first and the second polymer film layer (22, 24),
**characterized in that** the foil composite structure (20), rolled up, forms a lumen of the catheter, wherein overlapping regions of the foil composite structure (20) are sealingly interconnected by means of a bonded joining connection.

2. The catheter according to claim 1, wherein the joining connection does not contain a joining additive.

3. The catheter according to any one of the preceding claims, wherein the polymer foil (21) is formed from a thermoplastic plastic, in particular a liquid crystal polymer, a polyurethane, a polyamide, or a polyether block amide.

4. The catheter according to any one of the preceding claims, wherein a layer thickness of the polymer foil (21) is in the range of 10 to 100 µm.

5. The catheter according to any one of the preceding claims, wherein (i) the foil composite structure (20) includes a second polymer foil (23) and the conductor structure is embedded in an adhesive foil (26) connecting the first polymer foil (21) and the second polymer foil (23) or (ii) the conductor structure is covered with a lacquer layer.

6. The catheter according to any one of the preceding claims, wherein the conductor tracks (32) are made from copper.

7. The cathether according to any one of the preceding claims, wherein the electrodes (30) consist of a highly conductive, biocompatible metal material, in particular gold, platinum, platinum-iridium alloys or palladium alloys, or have a coating made from this metal material.

8. The cathether according to any one of the preceding claims, wherein at least one electrode (30) is designed as a ring electrode.

9. A method for producing a catheter, having the steps of:
a) providing a foil composite structure (20), which includes at least (i) one polymer foil (21), (ii) one or more electrodes (30) disposed on an outer surface of the foil composite structure (20), and (iii) a conductor structure having conductor tracks (32) for the electrical connection of the electrodes (30); and
b) forming the foil composite structure (20) in such a way that these overlap in such a way that this forms a lumen of the catheter, wherein an inner, relative to the catheter, polymer foil layer (22) and an outer, relative to the catheter, polymer foil layer (24) are produced, the electrodes are located at least partially on the outer surface, and the overlapping parts of the foil composite structure (20) are sealingly interconnected by bonded joining.

10. The method according to claim 9, wherein the bonded joining is carried out by fusion welding and without a joining additive.

11. The method according to claim 10, wherein the bonded joining is carried out by the local introduction of thermal energy by means of a heat source.

12. The method according to claim 10, wherein the bonded joining is carried out by means of ultrasonic welding.

13. The method according to claim 10, wherein the foil composite structure (20) comprises conductor tracks applied thereon or therein, which are disposed on the regions of the foil composite structure (20) to be fused thereon by means of fusion welding, and wherein these conductor tracks are used to electrically heat the areas to be fused thereon.

14. The method according to claim 9, wherein the bonded joining is carried out by partially dissolving areas of the foil composite structure (20) to be connected, using a solvent, by pressing the partially dissolved areas together and removing all the solvent.

## Revendications

1. Cathéter avec une structure composite en films (20) qui comprend au moins :
(i) un film de polymère (21) lequel est ainsi formé qu'une première couche de film de polymère (22) située à l'intérieur par rapport au cathéter et une deuxième couche de film de polymère (24) située à l'extérieur par rapport au cathéter sont créées ;
(ii) une ou plusieurs électrodes (30) disposées au moins partiellement sur une surface externe de la structure composite en films (20) ; et
(iii) une structure conductrice qui comprend des bandes conductrices (32) pour le branchement électrique des électrodes (30) et qui est disposée au moins partiellement entre les première et deuxième couches de films de polymère (22, 24),
**caractérisé en ce que** la structure composite en films (20) forme une lumière du cathéter à l'état enroulé, où des zones se chevauchant de la structure composite en films (20) sont reliées entre elles de manière étanche par une liaison de jonction par complémentarité des matières.

2. Cathéter selon la revendication 1, dans lequel la liaison de jonction ne contient aucun produit additif de jonction.

3. Cathéter selon l'une des revendications précédentes, dans lequel le film de polymère (21) est formé à partir d'une matière synthétique thermoplastique, notamment un polymère à cristal liquide, un polyuréthane, un polyamide ou un copolymère séquencé polyéther-amide.

4. Cathéter selon l'une des revendications précédentes, dans lequel l'épaisseur de couche de film de polymère (21) se situe dans un intervalle de 10 à 100 µm.

5. Cathéter selon l'une des revendications précédentes, dans lequel (i) la structure composite en films (20) comprend un deuxième film de polymère (23) et la structure conductrice est enfouie dans un film adhésif (26) reliant le premier film de polymère (21) et le deuxième film de polymère (23) ou (ii) la structure conductrice est recouverte d'une couche de laque.

6. Cathéter selon l'une des revendications précédentes, dans lequel les bandes conductrices (32) sont en cuivre.

7. Cathéter selon l'une des revendications précédentes, dans lequel les électrodes (30) sont constituées d'un matériau métallique hautement conducteur, biocompatible, notamment d'or, de platine, d'alliages platine-iridium ou d'alliages de palladium, ou présentent un revêtement à base de ce matériau métallique.

8. Cathéter selon l'une des revendications précédentes, dans lequel au moins une électrode (30) est conçue sous la forme d'une électrode annulaire.

9. Procédé de fabrication d'un cathéter, comprenant les étapes :
a) de mise au point d'une structure composite en films (20) qui comprend au moins (i) un film de polymère (21), (ii) une ou plusieurs électrodes (30) disposées sur une surface externe de la structure composite en films (20) et (iii) une structure conductrice avec des bandes conductrices (32) pour un branchement électrique des électrodes (30) ; et
b) de formation de la structure composite en films (20) de telle sorte que ceux-ci se chevauchent de telle manière qu'elle forme une lumière du cathéter, où une couche de films de polymère (22) se situant à l'intérieur par rapport au cathéter et une couche de films de polymère (24) se situant à l'extérieur par rapport au cathéter sont créées, les électrodes se trouvent au moins partiellement sur la surface à l'extérieur et les parties de la structure composite en films (20) se chevauchant sont reliées entre elles de manière étanche par une jonction par complémentarité des matières.

10. Procédé selon la revendication 9, dans lequel la jonction par complémentarité des matières est effectuée par un soudage par fusion et sans produit additif de jonction.

11. Procédé selon la revendication 10, dans lequel la jonction par complémentarité des matières est effectuée par un apport local d'énergie thermique au moyen d'une source de chaleur.

12. Procédé selon la revendication 10, dans lequel la jonction par complémentarité des matières est effectuée par un soudage par ultrasons.

13. Procédé selon la revendication 10, dans lequel la structure composite en films (20) présente des bandes conductrices rapportées dessus ou dedans qui sont disposées dans les zones de la structure composite en films (20) à faire fondre par un soudage par fusion, et dans lequel ces bandes conductrices servent au chauffage électrique des zones à faire fondre.

14. Procédé selon la revendication 9, dans lequel la jonction par complémentarité des matières est effectuée par une dissolution avec un solvant des zones de la structure composite en films (20) devant être reliées, par une compression des zones solubilisées en les assemblant et un enlèvement complet du solvant.
